## Europäisches Patentamt

(19) **European Patent Office**

**Office européen des brevets**

(11) Veröffentlichungsnummer: **0 040 331**
**B1**

(12) # EUROPÄISCHE PATENTSCHRIFT

(45) Veröffentlichungstag der Patentschrift:
04.04.84

(51) Int. Cl.³: **C 07 H 9/04,** C 25 B 3/02 //
C07D307/62

(21) Anmeldenummer: 81103028.7

(22) Anmeldetag: **22.04.81**

(54) **Verfahren zur Herstellung von Diacetonketogulonsäure.**

(30) Priorität: **21.05.80 DE 3019321**

(43) Veröffentlichungstag der Anmeldung:
**25.11.81 Patentblatt 81/47**

(45) Bekanntmachung des Hinweises auf die Patenterteilung:
**04.04.84 Patentblatt 84/14**

(84) Benannte Vertragsstaaten:
**CH DE FR GB LI**

(56) Entgegenhaltungen:
**DE - A - 2 460 156**

**CHEMICAL ABSTRACTS, Band 84, Nr. 1, 5. Januar 1976,
Seite 456, Nr. 5319e Columbus, Ohio, U.S.A.
CHEMICAL ABSTRACTS, Band 78, Nr. 1, 8. Januar 1973,
Seite 378, Nr. 4484u Columbus, Ohio, U.S.A.**

(73) Patentinhaber: **Merck Patent Gesellschaft mit
beschränkter Haftung, Frankfurter Strasse 250,
D-6100 Darmstadt (DE)**

(72) Erfinder: **Wittmann, Rolf, Dr., Tannenstrasse 6,
D-6109 Mühltal-Traisa (DE)**
Erfinder: **Wintermeyer, Willi, Dr., Gartenstrasse 4,
D-6104 Seeheim-Jugenheim 2 (DE)**
Erfinder: **Butzke, Jürgen, Mozartstrasse 3,
D-6110 Dieburg (DE)**

(74) Vertreter: **Schüttler, Reinhard, Dr., Merck Patent GmbH
Postfach 4119, D-6100 Darmstadt 1 (DE)**

Verfahren zur Herstellung von Diacetonketogulonsäure

Die Erfindung betrifft ein Verfahren zur Herstellung von Diacetonketogulonsäure, die ein wertvolles Zwischenprodukt bei der Herstellung von Vitamin C darstellt.

Bei der Herstellung von Diacetonketogulonsäure geht man in der Regel von Diacetonsorbose aus, die mit anorganischen Oxidationsmitteln wie HNO₃, H₂O₂, KMnO₄ oder insbesondere Hypochlorit zur Diacetonketogulonsäure oxidiert werden kann. Diese Verfahren haben jedoch den Nachteil, daß z. T. sehr große Mengen an anorganischen Salzen entstehen, die die Abwässer erheblich belasten. Besonderes Interesse hat daher in letzter Zeit die elektrochemische Oxidation gefunden, die dadurch besonders vorteilhaft ist, daß hierbei neben der anodischen Oxidation kathodisch Wasserstoff erzeugt wird, der im Rahmen der Gesamtsynthese von Vitamin C benötigt wird. Die elektrochemische Oxidation hat jedoch den Nachteil, daß ein ausreichend hoher Umsatz sehr lange Elektrolysezeiten und eine sehr große Elektrodenfläche erfordert. Durch die großen benötigten Elektrodenflächen und die, bedingt durch die langen Elektrolysezeiten, kurzen Standzeiten der Elektroden werden erhebliche Kosten verursacht. Außerdem besteht bei den sehr langen Elektrolysezeiten die Gefahr einer Weiteroxidation des Produktes, was sich in der bei der elektrochemischen Oxidation in der Regel niedrigeren Ausbeute gegenüber der naßchemischen Oxidation zeigt.

Die gleichen Nachteile ergeben sich bei der vom Prinzip her äußerst interessanten Oxidation mit Luft oder anderen sauerstoffhaltigen Gasen. Obwohl das unter Katalyse durch Edelmetalle wirkende Oxidationsmittel in praktisch unbegrenzter Menge zur Verfügung steht und deshalb preiswert eingesetzt werden kann, ergeben sich doch durch lange Reaktionszeiten und die dabei auftretende Weiteroxidation des Produkts erhebliche Nachteile.

Es bestand daher die Aufgabe, ein Verfahren zur Oxidation von Diacetonsorbose zu finden, bei dem diese Nachteile überwunden werden.

Es wurde nun gefunden, daß überraschenderweise diese Aufgabe dadurch gelöst werden kann, daß man die Oxidation mehrstufig durchführt und dabei unterschiedliche Oxidationsmittel verwendet.

Gegenstand der Erfindung ist daher ein Verfahren zur Herstellung von Diacetonketogulonsäure durch Oxidation von Diacetonsorbose, das dadurch gekennzeichnet ist, daß das Verfahren zweistufig durchgeführt wird, wobei in der ersten Stufe entweder elektrochemisch bis zu einem Oxidationsgrad von etwa 40—95% oder mit Luft bis zu einem Oxidationsgrad von etwa 20—95% oxidiert wird, und danach mit Hypochlorit durchoxidiert wird.

Die Vorteile dieses Verfahrens liegen insbesondere darin, daß durch die mehrstufige Fahrweise und die Verwendung unterschiedlicher Oxidationsmittel das Gesamtverfahren sehr viel besser optimiert werden kann. So kann z. B. die erste Teiloxidation im optimalen Bereich mit hohen Diacetonsorbosekonzentrationen geführt werden, wodurch in relativ kurzer Zeit ein hoher Umsatz erzielt werden kann, da erstaunlicherweise zunächst der Umsatz pro Zeiteinheit sehr hoch ist, wobei praktisch keine Weiteroxidation des Produktes erfolgt. Sobald dieser optimale Bereich verlassen wird, kann dann eine Oxidation mit einem zweiten Oxidationsmittel, vorzugsweise Hypochlorit, bis zum vollständigen Umsatz der Diacetonsorbose angeschlossen werden.

Sowohl die elektrochemische Oxidation von Diacetonsorbose als auch die Luftoxidation sind an sich bekannt. Das Verfahren der Luftoxidation, das z. B. in der deutschen Patentschrift 935 968, der ungarischen Patentschrift 162 772, der deutschen Offenlegungsschrift 2 123 621, der sowjetischen Patentschrift 137 913, in Liebigs Annalen der Chemie, Band 558, Seite 171 ff. (1947) und in Fortschritte der chemischen Forschung, Band 11, Seiten 285—374 (1969) beschrieben ist, wird im vorliegenden Verfahren analog angewendet. Die Oxidation wird jedoch nicht bis zum 100%igen Umsatz von Diacetonsorbose geführt, sondern schon bei einem Umsatz von 20—95%, vorzugsweise 30—80%, insbesondere 40—70%, abgebrochen und die Lösung wird der Restoxidation mit Hypochlorit zugeführt, wobei die gesamte noch verbliebene Diacetonsorbose umgesetzt wird.

Bei der elektrochemischen Oxidation geht man in der Regel von etwa 10—20 gew.-%igen Diacetonsorboselösungen aus, denen soviel Alkalihydroxid, insbesondere Natriumhydroxid zugegeben wird, daß das Reaktionsgemisch immer etwa 1,5—2 Mol Alkalihydroxid pro Mol Diacetonsorbose enthält.

Durch das Reaktionsgemisch, dem gegebenenfalls weitere Zusätze, wie z. B. die in den DE-AS 1 668 203 und DE-AS 2 410 034 beschriebenen Nickel- oder Eisensalze oder die in der DE-OS 2 505 911 beschriebenen Tenside, zugesetzt werden können, läßt man dann unter guter Durchmischung bei einer Temperatur von etwa 20—70° C, vorzugsweise 30—55°C einen Gleichstrom von etwa 0,2—20 A pro dm², vorzugsweise 0,5—10 A pro dm² Elektrodenoberfläche fließen. Die Zellenspannung beträgt dabei etwa 1,8—3,0 V, vorzugsweise etwa 2,0—2,7 V. Als Elektroden haben sich insbesondere Nickelelektroden bewährt. Die Elektrolyse kann sowohl kontinuierlich als auch diskontinuierlich in geteilten oder ungeteilten Zellen durchgeführt werden.

Im Gegensatz zu den bekannten Elektrolyseverfahren wird die Elektrolyse jedoch nicht bis zum 100%igen Umsatz von Diacetonsorbose geführt, sondern schon bei einem Umsatz von etwa 40—95%, vorzugsweise 60—85%, abgebrochen und die Lösung der Restoxidation mit Hypochlorit zugeführt, wobei die gesamte noch verbliebene Diacetonsorbose umgesetzt wird.

Diese Oxidation ist an sich bekannt. Allerdings wird bei dem erfindungsgemäßen Verfahren mit wesentlich niedrigeren Konzentrationen an Diacetonsorbose gearbeitet, da ja bereits ein Großteil der Ausgangsmenge zu Diacetonketogulonsäure oxidiert ist. Es war daher fraglich, ob die Hypochloritoxidation bei der geringen Restkonzentration an Diacetonsorbose von etwa 3—5 Gew.-% überhaupt noch anspringen würde. Überraschenderweise läuft die Reaktion jedoch glatt ab, wenn man bei einer Temperatur von etwa 40—80°C, vorzugsweise 50—70°C, und einem molaren Verhältnis von etwa 2—8 Mol, vorzugsweise etwa 3—5 Mol, Hypochlorit pro Mol Diacetonsorbose arbeitet. Als Katalysator wird ein Nickelsalz zugesetzt. Geeignet sind z. B. das Chlorid, Sulfat oder Nitrat. Bevorzugt wird Nickelchloridhexahydrat verwendet, das in einer Menge von etwa 5—150 g, insbesondere etwa 5—40 g pro kg Diacetonsorbose eingesetzt wird. Als Hypochlorit wird bevorzugt Natriumhypochlorit in Form einer wässerigen Lösung verwendet, die einen Gehalt von etwa 13—16 g Aktivchlor pro 100 ml besitzt.

Unter diesen Bedingungen ist die Restoxidation in etwa 1 Stunde beendet, worauf die Reaktionslösung in üblicher Weise aufgearbeitet werden kann. Dazu wird die Lösung filtriert, gekühlt und zur Fällung der Diacetonketogulonsäure mit Salzsäure angesäuert. Durch Abtrennung des kristallinen Produktes kann Diacetonketogulonsäure in einer Ausbeute von weit über 90% der Theorie gewonnen werden.

Überraschenderweise ist die bei dem erfindungsgemäßen Verfahren gewonnene Diacetonketogulonsäure wesentlich grobkristalliner als die bei der normalen Hypochloritoxidation erhaltene Diacetonketogulonsäure. Dieser vorteilhafte Effekt führt zu einem Produkt, das sich sehr viel leichter abtrennen und trocknen läßt und das danach sehr gut rieselfähig ist. In der folgenden Tabelle ist dieser überraschende Unterschied in der Korngrößenverteilung zahlenmäßig dargestellt.

| Korngröße | Hypochlorit-oxidation | Erfindungs-gemäßes Verfahren |
|---|---|---|
| <20 μm | 45% | 13% |
| 20—30 μm | 45% | 20% |
| 30—50 μm | 10% | 65% |
| 50—100 μm | — | 2% |
| >100 μm | — | — |

Die so erhaltene Diacetonketogulonsäure kann dann in üblicher Weise zu Ascorbinsäure weiterverarbeitet werden. Das erfindungsgemäße Verfahren stellt daher eine vorteilhafte Verbesserung der Gesamtascorbinsäuresynthese dar.

### Beispiel 1

Eine Mischung von 100 kg Diacetonsorboselösung (45gew.-%ig), 30 kg Natronlauge (50gew.-%ig), 0,001 kg Nonylphenolpolyglycoläther und 220 kg Wasser wird einem Elektrolyseur mit Nickelelektroden zugeführt und bei einer Temperatur von 50°C, beginnend mit einer Stromdichte von etwa 1000 A/m² elektrolysiert. Im Laufe der fortschreitenden Oxidation wird die Stromdichte bis auf 400 A/m² verringert. Beim Erreichen des Oxidationsgrades von ca. 77% der eingesetzten Diacetonsorbose wird die Elektrolyse beendet. Die ca. 340 kg Lösung mit einem Restgehalt von etwa 23% der eingesetzten Diacetonsorbose (entsprechend etwa 30 g/kg Lösung) werden mit 44 kg Natriumhypochloritlösung (enthaltend ca. 11,4 kg Aktivchlor) und 0,92 kg Nickelchloridlösung (enthaltend ca. 24% $NiCl_2 \cdot 6 H_2O$) versetzt und bei einer auf ca. 70°C ansteigenden Temperatur etwa 1 Stunde oxidiert. Danach wird die Lösung filtriert, auf etwa 0—5°C gekühlt und mit Salzsäure auf einen pH-Wert von etwa 1—2 angesäuert. Die dabei ausfallende Diacetonketogulonsäure wird abgetrennt und an der Luft getrocknet. Man erhält 46,5 kg (92,5% d. Th.) Diacetonketogulonsäure.

### Beispiel 2

3 kg einer etwa 13gew.-%igen Diacetonsorboselösung werden elektrochemisch zu 80,3% teiloxidiert und danach mit 685 ml Natriumhypochloritlösung, die 92,9 g Aktivchlor enthält und 11 ml einer ca. 24gew.-%igen Nickelchloridhexahydratlösung versetzt und bei einer Temperatur von etwa 60—70°C solange gerührt, bis kein Hypochlorit mehr nachweisbar ist. Nach der Aufarbeitung wie in Beispiel 1 werden 408 g (92,4% der Theorie) Diacetonketogulonsäure erhalten.

### Beispiel 3

1 kg einer etwa 13gew.-%igen Diacetonsorboselösung wird zu 83,5% elektrochemisch teiloxidiert und danach mit 182,2 ml Natriumhypochloritlösung, die 14,2 g Aktivchlor pro 100 ml enthält und 3,6 ml einer ca. 24gew.-%igen Nickelchloridhexahydratlösung durchoxidiert. Nach der üblichen Aufarbeitung erhält man 136,8 g (93,6% der Theorie) Diacetonketogulonsäure.

### Beispiel 4

6,83 kg einer etwa 13gew.-%igen Diacetonsorboselösung wird zu 95,3% elektrochemisch teiloxidiert und danach mit 500 ml Natriumhypochloritlösung, die 75,2 g Aktivchlor enthält und 21 ml einer ca. 24gew.-%igen Nickelchloridhexahydratlösung durchoxidiert. Man erhält 929 g (90,6% der Theorie) Diacetonketogulonsäure.

### Beispiel 5

1 kg einer etwa 13gew.-%igen Diacetonsorboselösung wird zu 64,6% elektrochemisch teiloxidiert und danach mit 254 ml Natriumhypochloritlösung, die 39,2 g Aktivchlor enthält und 12 ml einer ca. 24gew.-%igen Nickelchloridhexahydratlösung durchoxidiert. Nach üblicher Aufarbeitung erhält man 133 g (91% der Theorie) Diacetonketogulonsäure.

### Beispiel 6

In eine Lösung von 1,04 kg Diacetonsorbose in 7 l Wasser, die mit Natronlauge auf einen pH-Wert von 9—10 eingestellt ist und mit 50 g Platin-Aktivkohle (10% Pt) versetzt ist, wird bei einer Temperatur von 80—90° C während 6 Stunden Luft eingeleitet, wobei durch Zugabe von Natronlauge der pH-Wert konstant unterhalb von 10 gehalten wird. Die dabei zu 62% oxidierte Lösung wird durch Filtration vom Katalysator getrennt, mit der Restmenge von insgesamt 0,7 kg 50gew.-%iger Natronlauge versetzt und bei 50° C mit 18 ml einer 28,5gew.-%igen Nickelchloridhexahydratlösung versetzt. Durch Zugabe von 2,6 kg Hypochloritlösung, die ca. 340 g Aktivchlor enthält, wird die verbliebene Diacetonsorbose oxidiert. Nach üblicher Aufarbeitung erhält man 1,07 kg (91,6% der Theorie) Diacetonketogulonsäure.

### Patentansprüche

1. Verfahren zur Herstellung von Diacetonketogulonsäure durch Oxidation von Diacetonsorbose, dadurch gekennzeichnet, daß das Verfahren zweistufig durchgeführt wird, wobei in der ersten Stufe entweder elektrochemisch bis zu einem Oxidationsgrad von etwa 40—95% oder mit Luft bis zu einem Oxidationsgrad von etwa 20—95% oxidiert wird, und danach mit Hypochlorit durchoxidiert wird.

2. Verfahren zur Herstellung von Ascorbinsäure, dadurch gekennzeichnet, daß als Zwischenprodukt die nach Anspruch 1 hergestellte Diacetonketogulonsäure verwendet wird.

### Claims

1. Process for the preparation of diacetoneketogulonic acid by oxidation of diacetonesorbose, characterized in that the process is carried out in two steps, whereby in the first step it is oxidized either electrochemically to a degree of oxidation of about 40—95% or with air to a degree of oxidation of about 20—95%, and is then oxidized completely with hypochlorite.

2. Process for the preparation of ascorbic acid, characterized in that the diacetoneketogulonic acid prepared according to Claim 1 is used as an intermediate product.

### Revendications

1. Procédé de préparation d'acide diacétonecétogulonique par oxydation de diacétonesorbose, caractérisé en ce qu'on conduit le procédé en deux étapes, en oxydant dans la première étape soit électrochimiquement jusqu'à un degré d'oxydation d'environ 40—95%, soit à l'air jusqu'à un degré d'oxydation d'environ 20—95%, puis en ce qu'on oxyde à fond avec de l'hypochlorite.

2. Procédé de preparation d'acide ascorbique, caractérisé en ce qu'on utilise comme produit intermèdiaire l'acide diacétonecétogulonique préparé selon la revendication 1.